# EUROPEAN PATENT APPLICATION

(11) **EP 4 019 534 A1**
(43) Date of publication of application: **29.06.2022**
(21) Application number: 20216485.1
(22) Date of filing: 22.12.2020
(51) Int. Cl.: C07K 14/245, C12N 15/52

(54) **MICROBIAL CELLS POSSESSING A REDUCED LACTOSE INTERNALIZATION FOR PRODUCING AN OLIGOSACCHARIDE**

(71) Applicant: Chr. Hansen HMO GmbH, 53619 Rheinbreitbach (DE)
(72) Inventor: FRERIGMANN, Henning, 53619 Rheinbreitbach (DE); TRÖTSCHEL, Christian, 53619 Rheinbreitbach (DE); WARTENBERG, Dirk, 53619 Rheinbreitbach (DE); JENNEWEIN, Stefan, 53619 Rheinbreitbach (DE)

(57) **Abstract**

Disclosed are genetically engineered microbial cells for the production of an oligosaccharide of interest, wherein said microbial cells possess a variant of the *E. coli* lactose permease LacY which exhibits a reduced transport activity for lactose, and methods for producing an oligosaccharide by using said genetically engineered microbial cells.

SEQUENCE LISTING

<110>  Jennewein Biotechnologie GmbH

<120>  Microbial cells possessing a reduced lactose internalization for
       producing an oligosaccharide

<130>  P 2003 EP

<160>  2

<170>  PatentIn version 3.5

<210>  1
<211>  417
<212>  PRT
<213>  Escherichia coli

<400>  1

<210>  2
<211>  1254
<212>  DNA
<213>  Escherichia coli

<400>  2
atgtactatt taaaaaacac aaacttttgg atgttcggtt tattcttttt cttttacttt      60

tttatcatgg gagcctactt cccgtttttc ccgatttggc tacatgacat caaccatatc     120

agcaaaagtg atacgggtat tatttttgcc gctatttctc tgttctcgct attattccaa     180

ccgctgtttg gtctgctttc tgacaaactc gggctgcgca aatacctgct gtggattatt     240

accggcatgt tagtgatgtt tgcgccgttc tttattttta tcttcgggcc actgttacaa     300

tacaacattt tagtaggatc gattgttggt ggtatttatc taggcttttg ttttaacgcc     360

ggtgcgccag cagtagaggc atttattgag aaagtcagcc gtcgcagtaa tttcgaattt     420

ggtcgcgcgc ggatgtttgg ctgtgttggc tgggcgctgt gtgcctcgat tgtcggcatc     480

atgttcacca tcaataatca gtttgttttc tggctgggct ctggctgtgc actcatcctc     540

gccgttttac tctttttcgc caaaacggat gcgccctctt ctgccacggt tgccaatgcg     600

gtaggtgcca accattcggc atttagcctt aagctggcac tggaactgtt cagacagcca     660

aaactgtggt ttttgtcact gtatgttatt ggcgtttcct gcacctacga tgtttttgac     720

caacagtttg ctaatttctt tacttcgttc tttgctaccg gtgaacaggg tacgcgggta     780

tttggctacg taacgacaat gggcgaatta cttaacgcct cgattatgtt ctttgcgcca     840

ctgatcatta atcgcatcgg tgggaaaaac gccctgctgc tggctggcac tattatgtct     900

gtacgtatta ttggctcatc gttcgccacc tcagcgctgg aagtggttat tctgaaaacg     960

ctgcatatgt ttgaagtacc gttcctgctg gtgggctgct ttaaatatat taccagccag    1020

tttgaagtgc gtttttcagc gacgatttat ctggtctgtt tctgcttctt taagcaactg    1080

gcgatgattt ttatgtctgt actggcgggc aatatgtatg aaagcatcgg tttccagggc    1140

gcttatctgg tgctgggtct ggtggcgctg ggcttcacct taatttccgt gttcacgctt    1200

agcggccccg gcccgctttc cctgctgcgt cgtcaggtga atgaagtcgc ttaa          1254

## Description

The present invention relates to the internalization of lactose by microbial cells. More specifically, the present invention relates to the phenomenon of lactose killing, and provides means and methods for reducing or alleviating lactose killing of microbial cells. The present invention also relates to the advantages of using microbial cells being resistant to lactose killing in the production of oligosaccharides and glycoconjugates.

### Background

Carbohydrates (also called saccharides) are biomolecules that are classified in four chemical groups: monosaccharides, disaccharides, oligosaccharides and polysaccharides. Carbohydrates may be present in free form or in form of glycoconjugates when linked to a protein or polypeptide, then called glycoprotein, or to a lipid (then called glycolipid). Another classification of carbohydrates is based on their physiological effects. Carbohydrates which provide the human body with monosaccharides are defined as "digestible" carbohydrates, wherein "non-digestible" carbohydrates resist digestion in the humans' small intestine.

Oligosaccharides constitute a diverse group of molecules among the carbohydrates. As used herein, the term "oligosaccharide" typically refers to a polymeric saccharide molecule consisting of at least three monosaccharide moieties, but of no more than 12 monosaccharide moieties, preferably of no more than 10 monosaccharide moieties, that are linked to one another by glycosidic bonds. An oligosaccharide may consist of a linear chain of monosaccharide moieties, or may be a branched molecule, wherein at least one monosaccharide moiety has at least three monosaccharide moieties bound to it by glycosidic bonds. The monosaccharide moieties of an oligosaccharide can be selected from the group consisting of aldoses (e.g. arabinose, xylose, ribose, desoxyribose, lyxose, glucose, idose, galactose, talose, allose, altrose, mannose), ketoses (e.g. ribulose, xylulose, fructose, sorbose, tagatose), deoxysugars (e.g. rhamnose, fucose, quinovose), deoxy-aminosugars (e.g. *N*-acetylglucosamine, *N*-acetyl-mannosamine, *N*-acetyl-galactosamine), uronic acids (e.g. galacturonsäure, glucuronsäure) and ketoaldonic acids (e.g. *N-*acetylneuraminic acid).

Non-digestible oligosaccharides having the ability to be selectively metabolized by bifidobacteria and/or lactobacilli are designated as prebiotics. The benefits of consuming prebiotic oligosaccharides include relieving constipation, reducing the risk of getting colon cancer, inhibiting pathogens in gastrointestinal tract, increasing mineral absorption, modulating the immune system, producing short chain fatty acids and vitamins, reducing levels of blood cholesterol and lipids, and improving the microbial balance in the gut. A particular group of prebiotic oligosaccharides are the so-called "Human Milk Oligosaccharides" (HMOs).

Human Milk Oligosaccharides constitute a complex mixture of non-digestible oligosaccharides that is present in human milk. Human milk is unique with respect to its oligosaccharide content and composition. To date, more than 150 structurally distinct HMOs have been identified. The vast majority of HMOs is characterized by a lactose moiety at their reducing end. Many HMOs contain a fucose moiety and/or a sialic acid moiety at their non-reducing end. More generally speaking, the monosaccharides from which HMOs are derived are D-glucose, D-galactose, *N*-acetylglucosamine, L-fucose and *N*-acetylneuraminic acid.

Prebiotic oligosaccharides can be obtained from natural sources such as chicory and artichoke, or they can be produced by means of chemical synthesis or by enzymatic synthesis *in vitro.* For industrial scale production of oligosaccharides, in particular of some HMOs, fermentative methods were developed wherein an oligosaccharide of interest is produced by genetically engineered microbial cell, because HMOs can hardly be obtained from their natural sources. Industrial scale fermentative production of individual HMOs made these prebiotic compounds accessible for food industry, such that HMOs become available as supplements for infant formula.

In the fermentative production of oligosaccharides, i.e. in the production of an oligosaccharide of interest, in particular of an HMO, genetically engineered bacteria are typically cultivated in the presence of (i) an exogenous carbon source, and (ii) exogenous lactose. The term "exogenous" as used herein refers to compounds that are present in and/or added to the culture medium of the genetically engineered bacteria prior to and/or during cultivation of the genetically engineered bacteria for producing the oligosaccharide of interest. Typically, the compound as such is added to the culture medium, but in other embodiments, the compound can also be produced by a microbial cell that is present in and/or has been added to the culture medium. Lactose constitutes the disaccharide moiety at the reducing end of the HMO, and is typically the starting molecule for having individual HMOs synthesized intracellularly be the genetically engineered bacteria.

Lactose (β-D-galactopyranosyl-(1→4)-D-glucose) is a naturally occurring sugar that is found in milk of dairy animals. Lactose is a disaccharide composed of a glucose moiety and a galactose moiety. Infant mammals nurse on their mothers to drink milk, which is rich in lactose. In the intestine of the infant, the enzyme lactase (β-D-galactosidase) hydrolyzes lactose into its constituents, the monosaccharides glucose and galactose, which can be absorbed. Many enteric bacteria, including *Escherichia coli,* prefer glucose as a carbon source, and can effectively digest lactose if glucose is not available.

For intracellular biosynthesis of an oligosaccharide of interest bearing a lactose moiety at its reducing end by a microbial cell, it is desired to establish an intracellular lactose pool such that the lactose is not or not excessively degraded into its monosaccharides but becomes available for said oligosaccharide biosynthesis. One option of providing an intracellular lactose pool is the deletion or functional inactivation of the bacteria's endogenous gene(s) which encode enzymes which degrade or convert lactose. Exemplary genes are *lacZ* and/or *lacA* in *E*. *coli.* The *lacZ* gene encodes a β-galactosidase (LacZ) which hydrolyzes lactose, whereas *lacA* encodes a β-galactoside transacetylase (LacA), an enzyme that transfers an acetyl group from acetyl-CoA to β-galactosides.

However, increasing the intracellular lactose pool in a microbial cell is associated with drawbacks. One such drawback is a phenomenon called lactose killing. Lactose killing is a peculiar phenomenon in which the vast majority of *E. coli* bacteria die when they are transferred from a lactose-limited growth medium to a lactose-containing growth medium due to the rapid internalization of lactose across the bacterial cell membrane. The phenomenon of lactose killing is of particular relevance in fermentative production of oligosaccharides bearing a lactose moiety at their reducing end - such as HMOs - using a fermentation scheme wherein exogenous lactose is supplied to the fermentation medium in high amounts, regardless of whether the fermentation medium contains lactose when the microbial cells are inoculated in said fermentation medium and/or lactose is added to the fermentation medium in the course of fermentation either continuously or in at least one bolus. As lactose killing will at least delay growth and productivity of the microbial cell culture in the fermenter when lactose is supplied to the fermentation medium, it is desirable to use microbial cells being resistant to lactose killing for fermentative production of an oligosaccharide or glycoconjugate of interest.

In an attempt to overcome the drawbacks of lactose killing for microbial production of specialty products, WO 2016/075243 A1 suggests to render microorganisms resistant to lactose killing by altering the expression of a lactose transporter within a microorganism such that the expression level of said lactose transporter leads to a microorganism that withstands a lactose challenge and retains at least 50 % of the lactose influx obtained with a wild type expression cassette of said lactose transporter.

However, reduced expression levels of a lactose permease such as LacY in a bacterial cell may affect growth of the bacterial cells as it is known that the lactose permease and enzyme III^{Glc} of the phosphoenolpyruvate:carbohydrate phosphor-transferase system, which also participates in the regulation of the *bgl* operon and other non-PTS sugar permeases expression, interact.

In view thereof, it is desired to develop microbial strains which resist lactose killing, but which are not affected by adverse effects due to a reduced expression level of their lactose permease.

It has surprisingly been found that expression of particular variants of a LacY lactose permease instead of the wild-type LacY in a microbial cell increases the resistance of the microbial cell to lactose killing although expression levels of LacY and its variants remained unaltered, and that microbial cells possessing such a LacY variant are useful in the fermentative production of oligosaccharides.

### Summary

In a first aspect, provided are variants of a lactose permease which possess a reduced transport activity for lactose as compared to the *E*. *coli* wild-type lactose permease LacY.

In a second aspect, provided are nucleic acid molecules comprising a nucleotide sequence which encodes a variant of the *E. coli* wild-type lactose permease which possesses a reduced transport activity for lactose as compared to the wild-type *E. coli* lactose permease LacY.

In a third aspect, provided are genetically-engineered bacterial cells, wherein said bacterial cells are able to internalize lactose and possess a variant of the *E. coli* wild-type lactose permease LacY which possesses a reduced transport activity for lactose as compared to the wild-type *E*. *coli* lactose permease LacY.

In a fourth aspect, provided is the use of the genetically engineered bacterial cell possessing a variant of the *E. coli* wild-type lactose permease LacY which possesses a reduced transport activity for lactose as compared to the wild-type *E. coli* lactose permease LacY for the production of an oligosaccharide of interest.

In a fifth aspect, provided is a method for producing an oligosaccharide of interest, wherein the oligosaccharide of interest is synthesized in a genetically engineered bacterial cell possessing a variant of the *E. coli* wild-type lactose permease LacY which possesses a reduced transport activity for lactose as compared to the wild-type *E. coli* lactose permease LacY.

In a sixth aspect, provided is a method of alleviating the effect of lactose killing in a bacterial cell, the method comprises replacing an endogenous lactose permease of the bacterial cell with a variant of the *E. coli* wild-type lactose permease LacY which possesses a reduced transport activity for lactose as compared to the wild-type *E. coli* lactose permease LacY.

In a seventh aspect, provided is a method for improving efficacy of intracellular oligosaccharide biosynthesis from internalized lactose, the method comprises the use of a genetically engineered bacterial cell having its endogenous lactose permease replaced by a variant of the *E*. *coli* wild-type lactose permease LacY which possesses a reduced transport activity for lactose as compared to the wild-type *E. coli* lactose permease LacY.

In an eighth's aspect, provided is a method for improving reliability of producing an oligosaccharide of interest in a bacterial cell, wherein the method comprises the use of a genetically engineered bacterial cell a host cell for intracellular biosynthesis of the oligosaccharide of interest, wherein said bacterial host cell has its endogenous lactose permease replaced by a variant of the *E*. *coli* wild-type lactose permease LacY which possesses a reduced transport activity for lactose as compared to the wild-type *E*. *coli* lactose permease LacY.

### Brief description of the drawings

- Fig. 1: represents the amino acid sequence of the *E. coli* strain K12 lactose permease LacY as present in the UniProt Knowledgebase Database (Entry Number P02920; Entry version 180; UniProt release 2020_05 of October 07, 2020), and also being present as SEQ ID No. 1 in the attached sequence listing.
- Fig. 2: is a schematic representation of the topology of *E*. *coli* strain K12 lactose permease LacY within the cytoplasmic membrane of the *E. coli* cell as adapted from Sondej, M. and Seok, Y.-J., Phosphotransferase System by Cysteine Scanning Mutagenesis of E. coli Lactose Permease; Proc. Natl. Acad. Sci. (1999) 96:7, 3525-3530.
- Fig. 3: is a column chart showing the relative expression levels of *E. coli* wild-type LacY and a variant of *E*. *coli* LacY possessing a reduced transport activity for lactose as compared to the *E*. *coli* wild-type lactose permease LacY in *E. coli.*
- Fig. 4: is a column graph illustrating the effect of a LacY variant possessing a reduced transport activity for lactose as compared to the *E*. *coli* wild-type lactose permease LacY on the growth of *E*. *coli* cells in the presence of different amounts of lactose.
- Fig. 5: shows column graphs illustrating the transport activity of *E*. *coli* wild-type lactose permease LacY and a variant thereof which possesses a reduced transport activity for lactose in an in vitro assay.
- Fig. 6: is a graph illustrating LNT productivity of bacterial strains expressing *E. coli* wild-type lactose permease LacY or a variant thereof which possesses a reduced transport activity for lactose.
- Fig. 7: is a graph illustrating the growth kinetics of bacterial cells expressing *E. coli* wild-type lactose permease LacY or a variant thereof which possesses a reduced transport activity for lactose during LNT production.
- Fig. 8: displays the hydrophobicity and predicted transmembrane domains of different lactose permease variants.
- Fig. 9: displays the hydrophobicity and predicted transmembrane domains of different lactose permease variants.
- Fig. 10: displays the hydrophobicity and predicted transmembrane domains of a lactose permease variant.

### Detailed description

It has surprisingly been found by the inventors that certain variants of the *Escherichia coli* lactose permease LacY exhibit a reduced transport activity for lactose as compared to the *Escherichia coli* wild-type lactose permease LacY, and that such variants can advantageously be used in the fermentative production of oligosaccharides which requires the addition of exogenous lactose to the culture medium as *E. coli* cells possessing such a variant of the *E. coli* LacY are less sensitive to lactose killing.

According to the first aspect, provided are variants of the *E. coli* wild-type lactose permease LacY which possess a reduced transport activity for lactose as compared to the wild-type *E*. *coli* LacY. The reduced transport activity of the variants causes a reduced level of lactose internalization by an *E*. *coli* cell possessing such a variant instead of the wild-type lactose permease LacY.

The *E*. *coli* lactose permease LacY is a membrane protein of the so-called major facilitator superfamily (E.C. 2.A. 1.5.1). LacY is a symporter which utilizes the proton gradient across the cell membrane for co-translocating β-galactosides and protons into the cell. Thus, LacY can transport lactose, melibiose, lactulose and the analog methyl-1-thio-β,D-galactopyranoside (TMG), but not sucrose or fructose. The kinetics of β-galactoside transport activity of *E*. *coli* wild-type LacY is provided in Table 1.

**Table 1: Kinetics of β-galactoside transport by E. coli wild-type LacY**

| **Substrate** | **K_{M}** (mM) | **Vₘₐₓ (nmol/min/mg)** |
|---|---|---|
| lactose | 0.62 | 191 |
| melibiose | 0.24 | 105 |
| lactulose | 0.24 | 49 |
| TMG | 0.54 | 180 |

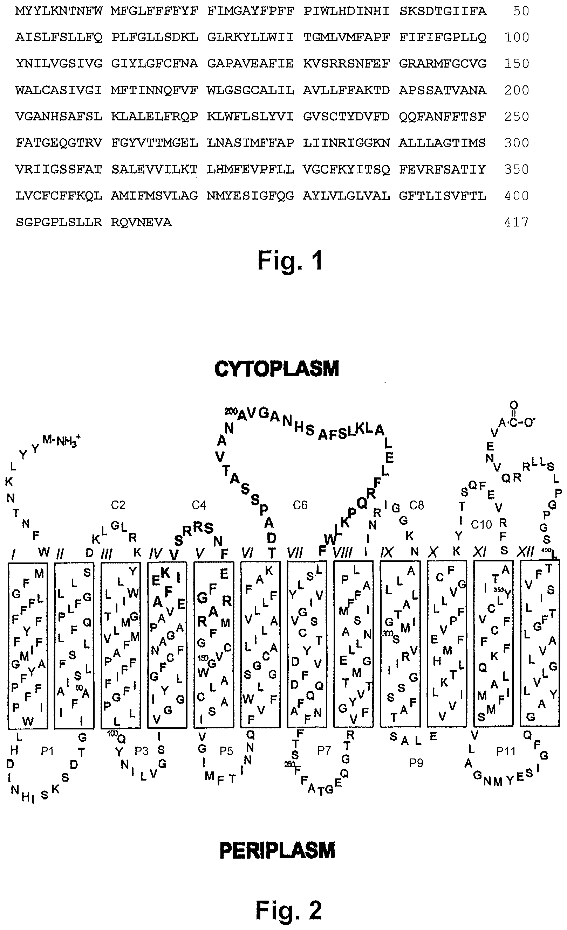

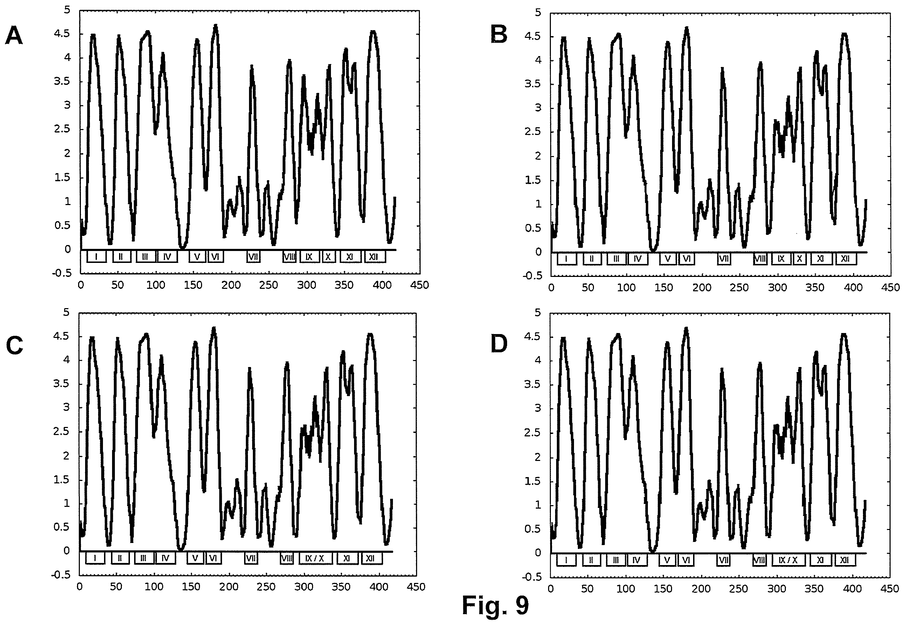

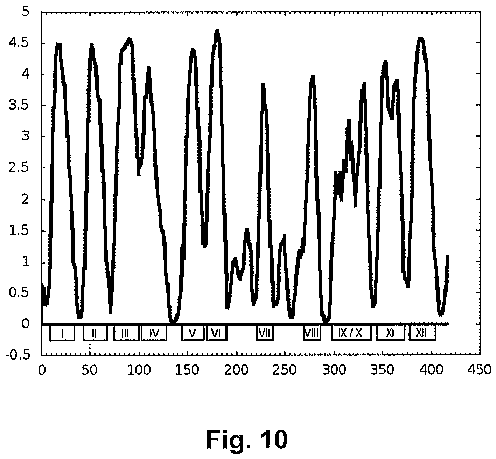

The *E. coli* lactose permease LacY has become a prototype in understanding the molecular details of membrane transport. Its amino acid sequence, i. e. the amino acid sequence of the "wild-type" *E. coli* LacY, is the amino acid sequence of LacY of *E. coli* strain K12 (UniProtKB - P02920, integrated into UniProtKB/Swiss-Prot database on July 21, 1986, last modified on June 17, 2020) as shown in Fig. 1 and as represented by SEQ ID No. 1. The *E*. *coli* LacY is a protein consisting of an amino acid chain of 417 amino acid residues in length, and has a calculated mass of 46,503 Da. *E*. *coli* wild-type LacY comprises 12 transmembrane domains as predicted by the Membrane Protein Secondary Structure Prediction server SPLIT 4.0 (splitbioinf.pmfst.hr/split/4/) as can be inferred from Fig. 9 A and Fig.10 A showing the transmembrane helix preference and the predicted transmembrane helix positions in the boxes below the graph. A more detailed topology of *E*. *coli* wild-type LacY is provided in Table 2 and shown in Fig. 2.

Mutagenic analysis of *E. coli* LacY revealed that some amino acid residues are irreplaceable for maintaining functionality of lactose transport. These amino acid residues and their function in lactose transport are provided in Table 3.

**Table 2: Topology of E. coli wild-type LacY**

| **amino acid position(s)** | **domain description** |
|---|---|
| 1 - 10 | cytoplasmic amino-terminal end |
| 11 - 33 | transmembrane helix I |
| 34 - 46 | periplasmic loop P1 |
| 47 - 67 | transmembrane helix II |
| 68 - 74 | cytoplasmic loop C2 |
| 75 - 99 | transmembrane helix III |
| 100 - 108 | periplasmic loop P3 |
| 109 - 131 | transmembrane helix IV |
| 132 - 138 | cytoplasmic loop C4 |
| 139 - 157 | transmembrane helix V |
| 158 - 167 | periplasmic loop P5 |
| 168 - 188 | transmembrane helix VI |
| 189 - 224 | cytoplasmic loop C6 |
| 225 - 246 | transmembrane helix VII |
| 247 - 259 | periplasmic loop P7 |
| 260 - 281 | transmembrane helix VIII |
| 282 - 290 | cytoplasmic loop C8 |
| 291 - 310 | transmembrane helix IX |
| 311 - 314 | periplasmic loop P9 |
| 315 - 334 | transmembrane helix X |
| 335 - 346 | cytoplasmic loop C10 |
| 347 - 366 | transmembrane helix XI |
| 367 - 379 | periplasmic loop P11 |
| 380 - 399 | transmembrane helix XII |
| 400 - 417 | cytoplasmic carboxy-terminal end |

**Table 3: Irreplaceable amino acid residues in the amino acid sequence of E. coli LacY**

| **position** | **amino acid** | **description** |
|---|---|---|
| 126 | E | substrate binding |
| 144 | R | substrate binding |
| 269 | E | substrate binding and proton translocation |
| 302 | R | proton translocation |
| 322 | H | proton translocation |
| 325 | E | proton translocation |

Mutagenic analysis of *E. coli* LacY also revealed that certain amino acid residues appear not essential for lactose transport. These non-essential amino acid residues are identified in Table 4.

**Table 4: Mutagenesis results on transport activity of E. coli LacY**

| **amino acid position** | **amino acid exchange** | **description** |
|---|---|---|
| 65 | L → V | No change in transport activity |
| 96 | G → A | No change in transport activity |
| 122 | A → S | No change in transport activity |
| 237 | D → N | Loss of activity |
| 264 | V → A | No change in transport activity |
| 279 | A → S | No change in transport activity |
| 355 | C → Q | No change in transport activity |
| 358 | K → T | Loss of activity |
| 367 | V → A | Increases transport of melibiose and impairs transport of TMG |

The variants of the *E. coli* lactose permease which possess a reduced transport activity for lactose as compared to *E. coli* wild-type lactose permease LacY exhibit a reduced internalization of lactose as compared to the *E. coli* wild-type lactose permease LacY expressed in the same *E*. *coli* strain at essentially the same level. The level of internalization of lactose by the variants of the *E*. *coli* lactose permease is reduced by at least 25 %, at least 30 %, at least 40 %, at least 50 %, at least 60 %, at least 70 %, at least 80 % or at least 90 % as compared to the level of lactose internalization provided by the *E. coli* wild-type lactose permease. In some embodiments, the level of internalization of lactose by the variants of the *E*. *coli* lactose permease LacY is reduced by about 30 % at an exogenous lactose concentration of 5 mM as compared to *E. coli* wild-type LacY. The term "about 30 %" is understood as between 26 %, 27 %, 28 % or 29 % at the lower end, and 34 %, 33 %, 32 % or 31 % at the upper end of the range.

The variant of the *E. coli* LacY possessing a reduced transport activity for lactose has at least 70 %, at least 80 %, at least 85 %, at least 90 %, at least 91 %, at least 92 %, at least 93 %, at least 94 %, at least 95 %, at least 96 %, at least 97 %, at least 98 %, or at least 99 % sequence identity to the *E. coli* wild-type LacY as compared to the amino acid sequence as represented by SEQ ID No. 1.

In additional and/or alternative embodiments, the variant of *E*. *coli* LacY which possesses a reduced transport activity of lactose comprises at least one amino acid substitution in transmembrane helix IX of *E*. *coli* wild-type lactose permease LacY. In some embodiments, the amino acid substitution in transmembrane helix IX of LacY is a substitution of each of one or more neutral amino acid residue in transmembrane helix IX with a basic amino acid residue.

In particular embodiments, the amino acid substitution in transmembrane helix IX comprises or consists of the replacement of at least one of the leucine residues (Leu, L) with a basic amino acid residue. Said leucine residues in transmembrane helix IX are the leucine residues at positions 292, 293 and 294 with respect to *E. coli* wild-type LacY as shown in Fig. 1 and SEQ ID No. 1. The basic amino acid residue is an amino acid selected from the group consisting of arginine (Arg, R), lysine (Lys, K) and histidine (His, H).

In an additional and/or alternative embodiment, the variant of *E. coli* LacY possessing a reduced transport activity for lactose as compared to the *E. coli wild-*type LacY is selected from the group consisting of LacY(L292R), LacY(L292K), LacY(L292H), LacY(L293R), LacY(L293K), LacY(L293H), LacY(L294R), LacY(L294K), LacY(L294H), and variants thereof as set forth herein before provided that such variants possess a basic amino acid residue at at least one of the positions 292, 293 and 294 as compared to *E. coli* wild-type LacY. Thus, the variant of the *E. coli* wild-type LacY bears the amino acid substitution at the position with respect to *E*. *coli* wild-type LacY as indicated in the bracket.

In a preferred embodiment, the variant of the *E*. *coli* LacY possessing a reduced transport activity of lactose as compared to the *E*. *coli* wild-type LacY comprises the substitution of the leucine residue at position 293 (with reference to the *E*. *coli wild-*type LacY) with an arginine residue (designated as LacY(L293R)).

In additional and/or alternative embodiments, the variant of the *E. coli* LacY possessing a reduced transport activity of lactose as compared to the *E. coli wild-*type LacY displays an altered membrane topology as compared to the membrane topology of *E. coli* wild-type LacY when analyzed using the SPLIT 4.0 server (splitinfo.pmfst.hr/split/4/) in that the amino acid sequence representing periplasmic loop P9 which separates transmembrane domains IX and X from each other in *E*. *coli* wild-type LacY is no longer identified as a stretch of amino acids that separates transmembrane domains IX and X from each other such that the predicted topology of the variants displays a fusion of transmembrane domains IX and X to a single transmembrane domain. Thus, these variants display a total of eleven transmembrane domains that are separated from one another by non-transmembrane loops

The variant of the *E*. *coli* lactose permease LacY which possesses a reduced level of lactose internalization as compared to the *E. coli* wild-type LacY also comprises functional fragments of said variants, i. e. lactose permeases that are truncated at the N-terminal end and/or at the C-terminal end and/or exhibit deletions within the amino acid sequence when compared to the amino acid sequences as represented by SEQ ID No. 1, but which exhibit lactose transport activity. In some embodiments, the truncated versions comprise substitution of at least one of the leucine residues corresponding to L292, L293 and L294 in SEQ ID No. 1 with a basic amino acid residue. The basic amino acid residue is an amino acid selected from the group consisting of arginine (Arg, R), lysine (Lys, K) and histidine (His, H).

It has surprisingly been found that a variant of the *E. coli* LacY which comprises a replacement of each of one or more of the three leucine residues in the amino acid sequence of transmembrane helix IX with a basic amino acid residue leads to a reduced level of lactose internalization by the *E*. *coli* cell expressing said variant as compared to an *E. coli cell* expressing the *E. coli* wild-type LacY. However, lactose internalization is not abolished in bacterial cells expressing one of the variants of *E. coli* LacY described herein before.

In a second aspect, provided are nucleic acid molecules comprising a nucleotide sequence which encodes a variant of the *E. coli* lactose permease LacY, wherein said variant possesses a reduced level of lactose transport activity as compared to the *E*. *coli* wild-type LacY. A nucleotide sequence encoding the *E*. *coli* wild-type LacY is represented by SEQ ID No. 2. The nucleotide sequence encoding the variant of the *E. coli* lactose permease is a nucleotide sequence which encodes any one of the LacY variants described herein before.

In some embodiments, the nucleotide sequence encoding the variant of *E. coli* LacY comprises a codon selected from the group consisting of CGA, CGC, CGG, CGT, AGA, AGG, AAA, AAG, CAC and CAT instead of a codon encoding the leucine residue (i.e. CT(A/C/G/T) or TT(A/T)) for one or more of amino acid positions 292, 293 and 294 with respect to the amino acid sequence as represented by SEQ ID No. 1.

In an additional and/or alternative embodiment, the nucleotide sequence which encodes the variant of the *E*. *coli* lactose permease has a sequence identity of at least 80%, at least 85%, at least 90%, at least 95%, at least 97%, at least 98% or at least 99% to the nucleotide sequence as represented by SEQ ID No. 2.

In additional and/or alternative embodiments, the nucleic acid molecule comprises a nucleotide sequence which encodes a lactose permease that possesses a reduced transport activity as compared to *E*. *coli* wild-type LacY and which hybridizes under stringent conditions to a nucleic acid molecule comprising a nucleotide sequence as represented by SEQ ID No. 2 and/or by a variant of the nucleotide sequence set forth in SEQ ID NO: 2, wherein the codon encoding the amino acid residue at one or more of positions 292, 293 and 294 with respect to the amino acid sequence as represented by SEQ ID No. 1 is replaced with a codon encoding a basic amino acid residue.

The term "hybridizing" as it is used herein means hybridizing under conventional conditions, as they are described in Sambrook et al. (Molecular Cloning. A laboratory manual, Cold Spring Harbor Laboratory Press, 2nd edition, 1989), preferably under stringent conditions. Stringent hybridization conditions are for example: hybridizing in 4 X SSC at 65 °C and subsequent multiple washing in 0.1 X SSC at 65 °C for a total of 1 hour. Less stringent hybridization conditions are for example: hybridizing in 4 X SSC at 37 °C and subsequent multiple washing in 1 X SSC at room temperature. The term "stringent hybridization conditions" as used herein can also mean: hybridizing at 68 °C in 0.25 M sodium phosphate, pH 7.2, 7 % SDS, 1 mM EDTA and 1 % BSA for 16 hours and subsequent washing twice with 2 X SSC and 0.1 % SDS at 68 °C.

The nucleic acid molecule is a recombinant or synthetic nucleic acid molecule. The nucleic acid molecule may be present as a linear nucleic acid molecule such as an amplicon, for example obtained by polymerase chain reaction (PCR) or ligase chain reaction (LCR), a chromosome such as a yeast artificial chromosome, or it may be present as a circular nucleic acid molecule such as a plasmid, cosmid or bacterial artificial chromosome.

In additional and/or alternative embodiments, the nucleotide sequence encoding the variant of *E*. *coli* LacY possessing a reduced transport activity for lactose is operably linked to expression control sequences effecting transcription and/or translation of said nucleotide sequence encoding the lactose permease in a genetically engineered microbial cell which contains said nucleic acid molecule.

The term "operably linked" as used herein, refers to a functional linkage between the nucleotide sequence encoding the lactose permease and a second nucleotide sequence, the nucleic acid expression control sequence (such as promoter, operator, enhancer, regulator, array of transcription factor binding sites, transcripttional terminator, ribosome binding site), wherein the expression control sequence affects transcription and/or translation of the nucleic acid corresponding to the nucleotide sequence encoding the lactose permease. Accordingly, the term "promoter" designates DNA sequences which usually "precede" a gene in a DNA polymer and provide a site for initiation of the transcription into mRNA. "Regulator" DNA sequences, also usually "upstream" of (i.e., preceding) a gene in a given DNA polymer, bind proteins that determine the frequency (or rate) of transcriptional initiation. Collectively referred to as "promoter/ regulator" or "control" DNA sequence, these sequences which precede a selected gene (or series of genes) in a functional DNA polymer cooperate to determine whether the transcription (and eventual expression) of a gene will occur. DNA sequences which "follow" a gene in a DNA polymer and provide a signal for termination of the transcription into mRNA are referred to as transcription "terminator" sequences.

According to the third aspect, provided is a genetically-engineered microbial cell, preferably a genetically-engineered microbial cell for the manufacturing of an oligosaccharide of interest, wherein said microbial cell is able to internalize lactose, i.e. the microbial cell internalizes lactose when lactose present in the microbial cell's environment. The genetically-engineered micorbial cell possesses a variant of a lactose permease for internalization of lactose that possesses a reduced transport activity for lactose as compered to the *E*. *coli* wild-type lactose permease LacY.

In additional and/or alternative embodiments, the genetically-engineered microbial cell possesses a lactose permease as described herein before. Preferably, the lactose permease possessing a reduced transport activity for lactose as compared to the *E*. *coli* wild-type lactose permease LacY is a variant of the *E*. coli lactose permease LacY wherein at least one of the leucine residues in transmembrnae domainIX is replacced with a basic amino acid.

In additional and/or alternative mebodiments, the microbial cell contains a nucleic acid molecule comprising a nucleotide seqeunce which encdoes the lactose permease which possesses a reduced transport activity as compared to *E. coli wild-*type lactose permease LacY. The nucleic acid molecule comprising the nucleotide sequence which encodes the lactose permease which possesses a reduced transport activity as compared to *E*. *coli* wild-type lactose permease LacY is for expression of the lactose permease. For expression of the lactose permease which possesses a reduced transport activity as compared to *E*. *coli* wild-type lactose permease LacY, the nucleotide sequence of the nucleic acid molecule is operably linked to expression control sequences.

In some embodiments, the genetically-engineered microbial cell does not possess any other lactose permease than the lactose permease which possesses a reduced transport activity as compared to *E*. *coli* wild-type lactose permease LacY.

In some embodiments, the genetically engineered microbial cell comprises at least one metabolic pathway for intracellular biosynthesis of a nucleotide activated sugar. The nucleotide activated sugar may be GDP-fucose, CMP-NeuNAc, UDP-galactose, UDP-glucose, GDP-mannose, UDP-glucosamine or UDP-galactosamine, UDP-N-acetylgalactosamine, UDP-N-acetylglucosamine.

In some embodiments, the genetically engineered microbial cell comprises at least one glycosyltransferase for transferring a monosaccharide moiety from a nucleotide activated monosaccharide as donor substrate to an acceptor molecule. The glycosyltransferase may be selected from the group consisting of fucosyltransferases, sialyltransferases, mannosyltransferases, N-acetylglucosaminyltransferases, N-acetylgalactosaminyltransferases, galactosyltransferases. The acceptor molecule is selected from the group consisting of lactose and oligosaccharides bearing a lactose moiety at their reducing end.

According to the fourth aspect, provided is the use of a genetically-engineered microbial cell as described herein before for the production of an oligosaccharide of interest.

As used herein, the term "oligosaccharide of interest" refers to the oligosaccharide that is intended to be produced.

The term "oligosaccharide" as used herein refers to a saccharide molecule consisting of three to twenty monosaccharide residues, wherein each of said monosaccharide residues is bound to at least one other of said monosaccharide units by a glycosidic linkage. The oligosaccharide may be a linear chain of monosaccharide residues or a branched chain of monosaccharide residues.

In some embodiments, the oligosaccharide of interest is an oligosaccharide comprising a lactose moiety at its reducing end. In an additional and/or alternative embodiment, the desired oligosaccharide is a human milk oligosaccharide (HMO).

In an additional and/or alternative embodiment, the desired oligosaccharide is a HMO and/or oligosaccharide selected from the group consisting of 2'-fucosyllactose (2'-FL), 3-fucosyllactose (3-FL), 2',3-difucosyllactose (DFL), lacto-*N*-triose II, lacto-*N*-tetraose (LNT), lacto-*N*-neotetraose (LN*n*T), lacto-*N*-fucopentaose I (LNFP-I), lacto-*N*-*neo*fucopentaose I (LN*n*FP-I), lacto-*N*-fucopentaose II (LNFP-II), lacto-*N-*fucopentaose III (LNFP-III), lacto-*N*-fucopentaose V (LNFP-V), lacto-*N*-*neo*fucopentaose V (LN*n*FP-V), lacto-*N*-hexaose (LNH), lacto-*N*-neohexaose (LNnH), para-lacto-N-hexaose (paraLNH), para-lacto-N-neohexaose (paraLNnH), difucosyl-lacto-N-neohexaose (DF-LNnH), lacto-*N*-difucosylhexaose I, lacto-*N*-difucosylhexaose II, *para*-lacto-*N*-fucosylhexaose (paraLNH), fucosyl-lacto-*N*-sialylpentaose a (F-LST-a), fucosyl-lacto-*N*-sialylpentaose b (F-LST-b), fucosyl-lacto-*N*-sialylpentaose c (F-LST-c), fucosyl-lacto-*N*-sialylpentaose c, disialyl-lacto-*N*-fucopentaose, 3-fucosyl-3'-sialyllactose (3F-3'-SL), 3-fucosyl-6'-sialyllactose (3F-6'-SL), lacto-*N*-neodifuco-hexaose I, 3'-sialyllactose (3-SL), 6'-sialyllactose (6-SL), sialyllacto-*N*-tetraose a (LST-a), sialyllacto-*N*-tetraose b (LST-b), sialyllacto-*N*-tetraose c (LST-c), disialyl-lacto-*N*-tetraose (DS-LNT), Disialyl-lacto-*N*-fucopentaose (DS-LNFP V), lacto-N-neodifucohxaose I (LNnDFH I), 3'-galactosyllactose (3'-GL), 6'-galactosyllactose (6'-GL).

The genetically-engineered microbial host cell may be a prokaryotic cell or a eukaryotic cell. Suitable microbial host cells include yeast cells, bacterial cells, archaebacterial cells and fungal cells.

In an additional and/or alternative embodiment, the prokaryotic cell is a bacterial cell, preferably a bacterial cell selected from bacteria of a genus selected from the group consisting of *Bacillus, Bifidobacterium, Clostridium, Corynebacterium, Enterococcus, Lactobacillus, Lactococcus, Micrococcus, Micromonospora, Pseudomonas, Rhodococcus* and *Sporolactobacillus.* Suitable bacterial species are *Bacillus subtilis, B. licheniformis, B. coagulans, B. thermophilus, B. laterosporus, B. megaterium, B. mycoides, B. pumilus, B. lentus, B. cereus, B. circulans, Bifidobacterium longum, B. infantis, B. bifidum, Citrobacter freundii, Clostridium cellulolyticum, C. Ijungdahlii, C. autoethanogenum, C. acetobutylicum, Corynebacterium glutamicum, Enterococcus faecium, E. thermophiles, Escherichia coli, Erwinia herbicola* (*Pantoea agglomerans*)*, Lactobacillus acidophilus, L. salivarius, L. plantarum, L. helveticus, L. delbrueckii, L. rhamnosus, L. bulgaricus, L. crispatus, L. gasseri, L. casei, L. reuteri, L. jensenii, L. lactis, Pantoea citrea, Pectobacterium carotovorum, Proprionibacterium freudenreichii, Pseudomonas fluorescens, P. aeruginosa, Streptococcus thermophiles* and *Xanthomonas campestris.*

In an additional and/or alternative embodiment, the eukaryotic cell is a yeast cell, preferably a yeast cell selected from the group consisting of *Saccharomyces sp.,* in particular *Saccharomyces cerevisiae, Saccharomycopsis sp., Pichia sp.,* in particular *Pichia pastoris, Hansenula sp., Kluyveromyces sp., Yarrowia sp., Rhodotorula sp.,* and *Schizosaccharomyces sp.*

According to another aspect, provided are methods for the production of an oligosaccharide of interest, wherein the oligosaccharide of interest is synthesized in/by a genetically engineered microbial cell. Then methods comprise providing a genetically engineered microbial cell for the synthesis of the oligosaccharide of interest, wherein the genetically engineered microbial cell is a genetically engineered microbial cell as described herein before. The genetically engineered microbial cell is cultivated in a culture medium and under conditions that are suitable for the genetically engineered microbial cell to synthesize an oligosaccharide of interest. For the biosynthesis of the oligosaccharide of interest, the genetically engineered microbial cell is cultivated in the presence of exogenous lactose. The methods further comprise the step of recovering the oligosaccharide of interest that has been synthesized by the genetically engineered microbial cell. The oligosaccharide of interest is recovered from the genetically engineered microbial cell and/or from the culture medium.

According to another aspect, provided is a method for alleviating the effect of lactose killing of a microbial cell, the method comprises the step of replacing the endogenous lactose permease of the microbial cell with a lactose permease possessing a reduced transport activity for lactose as compared to *E*. *coli* wild-type lactose permease LacY. In some embodiments, the lactose permease possesses a reduced transport activity for lactose as compared to *E*. *coli* wild-type lactose permease LacY is a variant of the *E*. *coli* wild-type lactose permease as described herein before.

In some embodiments, the replacement of the endogenous lactose permease with a lactose permease with a reduced transport activity for lactose as compared to *E*. *coli* wild-type lactose permease LacY occurs by replacing the endogenous lactose permease gene(s) of the microbial cell with at least one gene encoding a lactose permease possessing a reduced transport activity for lactose as compared to *E*. *coli* wild-type lactose permease LacY.

Replacing the endogenous lactose permease of the microbial cell with a lactose permease possessing a reduced transport activity for lactose as compared to *E. coli* wild-type lactose permease LacY reduces or prevents killing of the microbial cell when exposed to exogenous lactose, in particular when exposed to high concentrations of exogenous lactose. The term "high concentrations of lactose" when used for assessing the microbial cells' resistance to lactose killing means a lactose concentration in the culture medium for cultivating the microbial cell of at least 5 mM, preferably about 10 mM, more preferably about 20 mM, and most preferably about 25 mM.

According to another aspect, provided is a method for improving the efficacy and/or reliability of producing an oligosaccharide of interest in or by a microbial cell, wherein the genetically engineered microbial cell has to be cultivated in the presence of exogenous lactose for a biosynthesis of the oligosaccharide of interest. The method comprises the step of providing a microbial cell as described herein before for the production of the oligosaccharide of interest. Said microbial cell possesses a variant of the *E*. *coli* lactose permease LacY which possesses a reduced transport activity for lactose. The microbial cell is able to synthesize the oligosaccharide of interest by internalizing exogenous lactose. The microbial cell is less sensitive to the addition / presence of exogenous lactose due to the reduced level of lactose internalization by the microbial cell as compared to a microbial cell possessing the *E*. *coli* lactose permease LacY. Therefore, cultivation of the microbial cell and/or production of the oligosaccharide by the microbial cell is less sensitive to the presence and/or addition of exogenous lactose.

Rendering the production of an oligosaccharide in or by a microbial cell more efficient and/or more reliable is of particular interest in large-scale / industrial production processes. The terms "large-scale" and "industrial" indicate that the production of the oligosaccharide of interest occurs by microbial fermentation in a volume of fermentation broth exceeding 100 L, 500 L, 1000 L, 5000 L, 10,000 L 50,000 L 100,000 L or even 200,000 L.

The present invention will be described with respect to particular embodiments and with reference to drawings, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventtive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

### Examples

### Example 1: LC/MS analytics

The identity of lacto-N-tetraose being produced was determined by multiple reaction monitoring (MRM) using an LC triple-quadrupole MS detection system (Shimadzu LCMS-8050). Precursor ions were selected and analyzed in quadrupole 1, followed by collision-induced fragmentation (CID) with argon and selection of fragment ions in quadrupole 3. Selected transitions and collision energies for intermediates and end-product metabolites are listed in Table 5.

**Table 5. List of diagnostic MRM transitions and parameters for the identification of LNT, (CE = Collision energy) of the Shimadzu LCMS-8050 Triple Quadrupole (QQQ) Mass Analyzer.**

| **Metabolite** | **Mode** | **Precursor m/z** | **Product m/z** | **Dwell time [msec]** | **Q1 pre bias (V)** | **CE** | **Q3 pre bias (V)** |
|---|---|---|---|---|---|---|---|
| LNT | - | 706.2 | 202.1 | 9 | 32 | 22 | 19 |
| | - | 706.2 | 142.0 | 9 | 32 | 31 | 23 |
| | - | 706.2 | 382.1 | 9 | 32 | 17 | 26 |

The oligosaccharide LNT in particle-free culture supernatants or cytoplasmic fractions were separated by high performance liquid chromatography (HPLC) using a Waters XBridge Amide HPLC column (3.5 µm, 2.1 Å 50 mm) for both neutral or acidic sugars. Neutral sugars were eluted with isocratic flow of H₂O with 0.1% (v/v) ammonium hydroxide. The HPLC system encompasses a Shimadzu Nexera X2 SIL-30AC_{MP} autosampler run at 8°C, a Shimadzu LC-20AD pump, and a Shimadzu CTO-20AC column oven running at 35°C for elution of neutral sugars and was adjusted to 50°C for elution of acidic sugars. For the analysis of LNT 1 µl was injected into the instrument. Flow rate was set 0.3 mL/min corresponding to a run time of 5 min. LNT was analyzed in negative ion mode. The mass spectrometer was operated at unit resolution. Collision energy, Q1 and Q3 pre-bias were optimized for each analyte. Quantification methods were established using commercially available standards (Carbosynth and Elicityl).

### Example 2: Minimal culture medium

The culture medium used to grow the cells for the production of the desired oligosaccharide contained: 3 g/L KH₂PO₄, 12 g/L K₂HPO₄, 5 g/L (NH₄)SO₄, 0.3 g/L citric acid, 0.1 g/L NaCl, 2 g/L MgSO₄ × 7 H₂O and 0.015 g/L CaCl₂ × 6 H₂O, supplemented with 1 ml/L trace element solution (54.4 g/L ammonium ferric citrate, 9.8 g/L MnCl₂ × 4 H₂O, 1.6 g/L CoCl₂ × 6 H₂O, 1 g/L CuCl₂ × 2 H₂O, 1.9 g/L MnCl₂ × 4 H₂O, 1.1 g/L Na₂MoO₄ × 2 H₂O, 1.5 g/L Na₂SeO₃, 1.5 g/L NiSO₄ × 6 H₂O). The pH of the medium was adjusted to 7.0. Lactose was added to the medium in a concentration of 10 mM to 25mM for synthesis of the desired oligosaccharide by the cells and for testing the cells' sensitivity to lactose.

### Example 3: Generating a lacto-N-tetraose producing E. coli strain with reduced lactose sensitivity

A metabolically engineering derivative of *E. coli* BL21(DE3) which was capable of producing lacto-*N*-tetraose (LNT) was obtained by genetic engineering. For reducing the sensitivity of said bacterial LNT production strain against lactose, the strain's endogenous *lacY* gene was modified by using mismatch oligonucleotides to generate the *lacY* variant *lacY*(L293R) which encodes the lactose permease LacY(L293R).

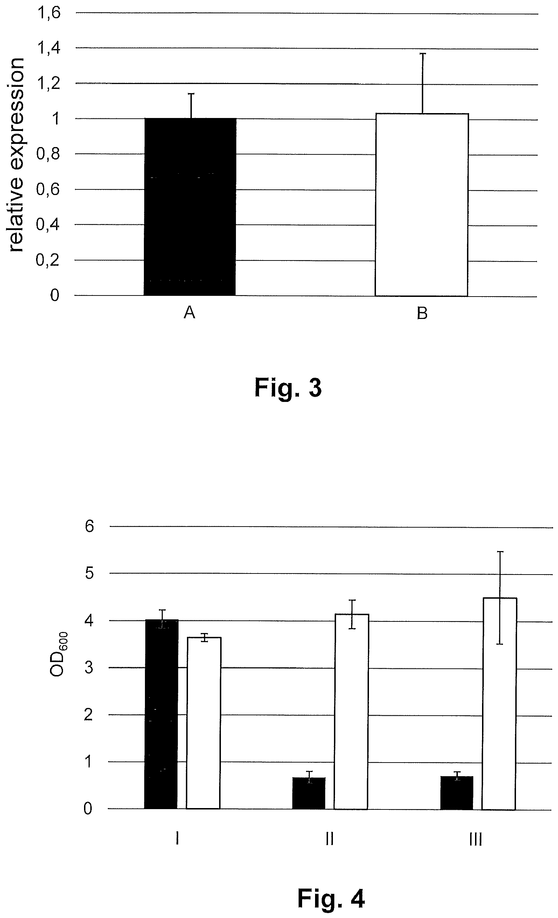

For expression analyses individual clones obtained from above-referenced mutagenesis approach were inoculated in 25 ml mineral salts medium in shaking flasks. Expression analysis of each clone was performed in 3 replicates. The bacterial cells were grown at 30°C and a relative humidity of 80% with constant agitation at 120 rpm. After 20 h of culturing, the optical densities of these precultures were determined and the main cultures were seeded to an OD₆₀₀ of 0.05 in 25 ml of mineral salts medium. The main cultures were cultured for 20 h under the same conditions as the pre-cultures were cultured. Then the bacterial cells were harvested by centrifugation and used for extraction of RNA and subsequent expression analysis by qPCR. The relative quantification of expression levels was performed using the comparative Δ cycle threshold method, the calculated relative expression values were normalized to *gapA* and compared with the expression level in the parental strain used as control. The results are shown in Fig. 3. Relative expression of LacY (Fig. 3, column A) was at the same level as relative expression of LacY(L293R) (Fig. 3, column B). Hence, introducing the mutation in the *lacY* gene which lead to the lactose permease variant LacY(L293R) did not alter expression of the respective lactose permease genes.

For testing lactose sensitivity of the bacterial LNT production strains bearing LacY(L293R), individual clones of said strain were inoculated in 25 ml mineral salts medium in shaking flasks. The bacterial cells were grown at 30 °C and 80 % relative humidity with constant agitation at 120 rpm. After 20 h culturing, the optical density of the pre-cultures was determined and the main cultures were inoculated to an OD₆₀₀ of 0.3 in 25 ml of mineral salts medium. Lactose was added to the culture medium two hours after inoculation of the cultures to final concentrations of 10 or 25 mM. The optical densities (OD₆₀₀) of the main cultures were determined 20 hours after addition of lactose.

The optical densities are shown in Fig. 4. This figure reveals that bacterial strains possessing LacY (black columns) exhibit a similar growth as bacterial strains possessing the lactose permease variant LacY(L293R) (white columns) in the absence of lactose in the culture medium (column subset I). In the presence of 10 mM lactose (column subset II) or 25 mM lactose (columns subset III), the growth of bacterial cells possessing LacY was severely reduced as compared to the growth in the absence of lactose, whereas the growth of bacterial cells possessing the lactose permease variant LacY(L293R) was not reduced in the presence of 10 mM or 25 mM lactose in their culture medium.

### Example 4: Quantification of lactose import using ¹⁴C-labelled lactose

For measuring lactose transport, *E*. *coli* strains expressing different variants of the *E. coli* lactose permease LacY were inoculated in 3 ml LB medium and cultured for 20 hours at 37 °C in a shaker. Bacterial cell pellets from these cultures were used to inoculate 100 ml minimal medium in shaking flasks. These cultures were incubated for 16 hours at 37 °C in a shaker. The bacterial cells were then sedimented by centrifugation and the resulting pellets were washed twice with fresh cold (4 °C) culture medium and finally resuspended in cold medium. The optical density (OD₆₀₀) of the cell suspension was set to 5 with cold medium, and the cell suspension is were stored on ice until use for the internalization assay.

Three minutes prior to the transport assay, the bacterial cells were placed in a 37 °C thermo block. The transport assays were started by addition of a mixture of ¹⁴C-labelled lactose and non-radiolabeled lactose to a final concentration of 0.5 mM lactose and 50,000 dpm. After 30 seconds incubation time the transport assay was stopped by separating the bacterial cells from their medium using a filter system comprising a nitrocellulose filter (0.22 µM pore size; Millipore, Eschborn, DE). The bacterial cells on the filter were washed twice with 100 mM LiCI and the filter was used subsequently to quantify remaining radioactivity in a Tricarb 2800 scintillation counter (Perkin Elmar, Waltham, MA). To this end, the filters were transferred into scintillation tubes and overlaid with 6 ml Rotiszint eco plus (Roth, Karlsruhe, DE).

In a second set of experiments, the transport assays were started by addition of a mixture of ¹⁴C-labelled lactose and non-radiolabeled lactose to a final concentration of 5 mM lactose and 550,000 dpm. The bacterial cells were collected by filtration after 60 seconds incubation, and washed twice with 3 ml medium containing 5 mM unlabelled lactose.

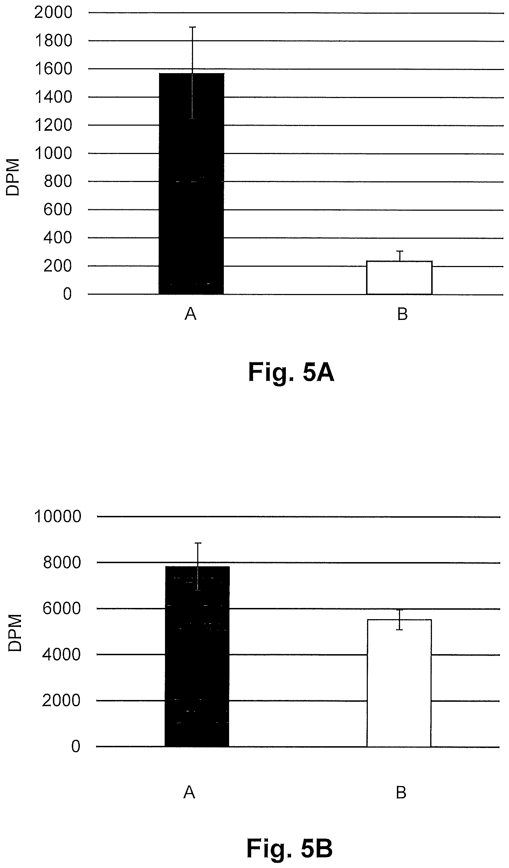

As can be seen in Fig. 5, bacterial cells possessing LacY (black column) had much more radioactivity incorporated than bacterial cells possessing the lactose permease variant LacY(L293R) (white column), wherein Fig. 5 A displays lactose internalization at an exogenous lactose concentration of 0.5 mM whereas Fig. 5 B displays lactose internalization at an exogenous lactose concentration of 5 mM. Thus, the lactose permease variant LacY(L293R) leads to a reduced but detectable lactose import into the bacterial cells as compared to LacY.

### Example 5: Lacto-N-tetraose production by metabolically engineered E. coli in a lab scale fermentation

The impact of reduced lactose import and reduced lactose sensitivity by a bacterial strain on its oligosaccharide production was analyzed in a small-scale fermentation.

Therefore, fermentative production of LNT by an *E. coli* strain bearing LacY and by an *E. coli* strain bearing LacY(L293R) were compared in a 3 litre scale.

In a first set of fermentations each of the bacterial strains was inoculated from a preculture in a fermentation broth containing 20 mM lactose. While the *E*. *coli* strain bearing LacY(L293R) did not display any problems in growth, the *E*. *coli stain* bearing wild-type LacY showed a severe delay in growth (data not shown) rendering this experimental approach unsuitable for comparing LNT production. Furthermore, the lactose level can be kept high in cultures of *E. coli* strain bearing LacY(L293R) by applying a continuous lactose feed (3.2 ml/L/h) without causing any setback in the OD₆₀₀ and without apparent lactose-caused cell lysis.

For comparing the LNT production of the different LNT production strains despite the severe delay of growth of bacteria possessing LacY in the presence of lactose, the strains were inoculated in culture medium lacking lactose. In these cultures, the lactose feed (2.5 ml/L/h) was started at the end of the batch phase. However, although lower lactose feed was used, a decline in OD₆₀₀ was observed after 40 hours in *E. coli* bearing LacY.

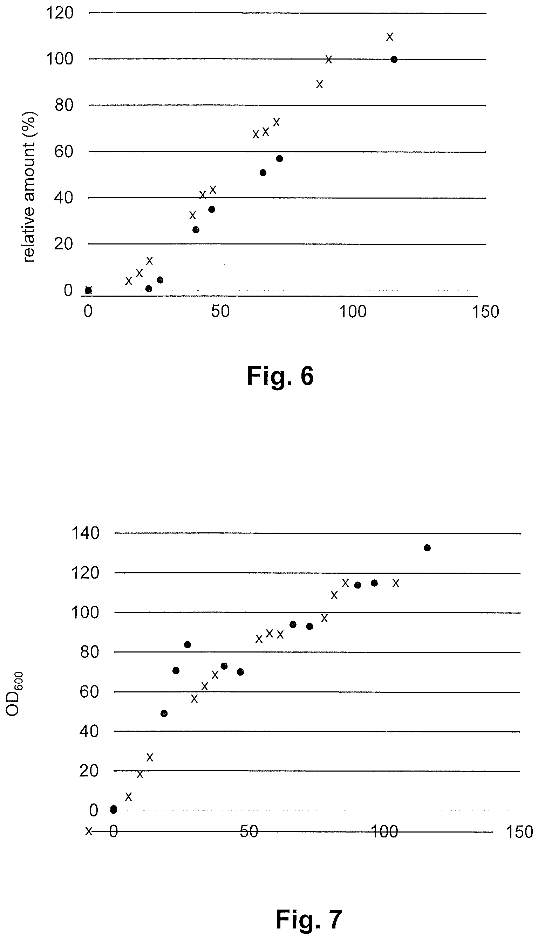

The results of the small-scale fermentation are represented in Figures 6 and 7. While Figure 6 displays the relative amount of LNT production of a bacterial strain bearing LacY (•) as compared to a bacterial strain bearing LacY(L293R) (x) instead of LacY, the growth of both strains in their culture media is shown in Fig. 7.

While growth of the LNT-producing strain bearing LacY (•) is significantly impaired after about 25 hours, the LNT-producing strain bearing LacY(L293R) (x) exhibits regular growth kinetics throughout the fermentation, reaching a higher optical density in the culture (Fig. 7).

Surprisingly, the yield of LNT obtained from the strain bearing LacY(L293R) (x) was higher than that obtained from the LNT-producing strain bearing LacY (•) as can be inferred from Fig. 6. The amount of LNT in the fermentation using the LNT-producing strain bearing LacY at 120 hours was set as 100 %.

Due to the ability of the *E. coli* strain bearing LacY(L293R) to cope with lactose in the batch medium, the strain can start earlier to produce LNT and is therefore capable to produce more LNT than the control strain bearing wild-type LacY. However, the biggest advantage of the reduced lactose sensitivity is the ability to circumvent the risk of cell lysis and potential total loss of the fermentation upon feeding lactose for the oligosaccharide production.

### Example 6: Determining membrane topology of E. coli LacY variants

The amino acid sequences of various variants of *E*. *coli* wild-type LacY were subjected to a prediction of their transmembrane secondary structures using the SPLIT 4.0 server (http://splitbioinf.pmfst.hr/split/4/) on and described in:
Juretic, D., Zoranic, L., Zucic, D. "Basic charge clusters and predictions of membrane protein topology" J. Chem. Inf. Comput. Sci. Vol. 42, pp. 620-632, 2002.
Juretic, D., Lee, B. K., Trinajstic, N., and Williams, R. W. "Conformational preference functions for predicting helices in membrane proteins." Biopolymers 33, 255-273 (1993).
Juretic, D., Lucic, B., Zucic, D. and Trinajstic, N. "Protein transmembrane structure: recognition and prediction by using hydrophobicity scales through preference functions." Theoretical and Computational Chemistry, Vol.5. Theoretical Organic Chemistry, pp. 405-445. Editor: Parkanyi, C., Elsevier Science, Amsterdam, 1998. (*SPLIT 3.1 results are described in that paper*)
Juretic, D., Zucic, D., Lucic, B. and Trinajstic, N. "Preference functions for prediction of membrane-buried helices in integral membrane proteins." Computers Chem. Vol. 22, No. 4, pp. 279-294, 1998. (*SPLIT 3.1 results are described in that paper as well*) Juretic, D. and Lucin A. "The preference functions method for predicting protein helical turns with membrane propensity." J. Chem. Inf. Comput. Sci. Vol. 38, No. 4, pp. 575-585, 1998. (*SPLIT 3.5 results are described in that paper*)
Juretic, D., Jeroncic, A. and Zucic, D. "Sequence analysis of membrane proteins with the web server SPLIT." Croatica Chemica Acta Vol. 72, No. 4, pp. 975-997, 1999.
Juretic, D., Jeroncic, A. and Zucic, D. "Prediction of initiation sites for protein folding." Periodicum Biologorum 101, No 4, 339-347, 1999.

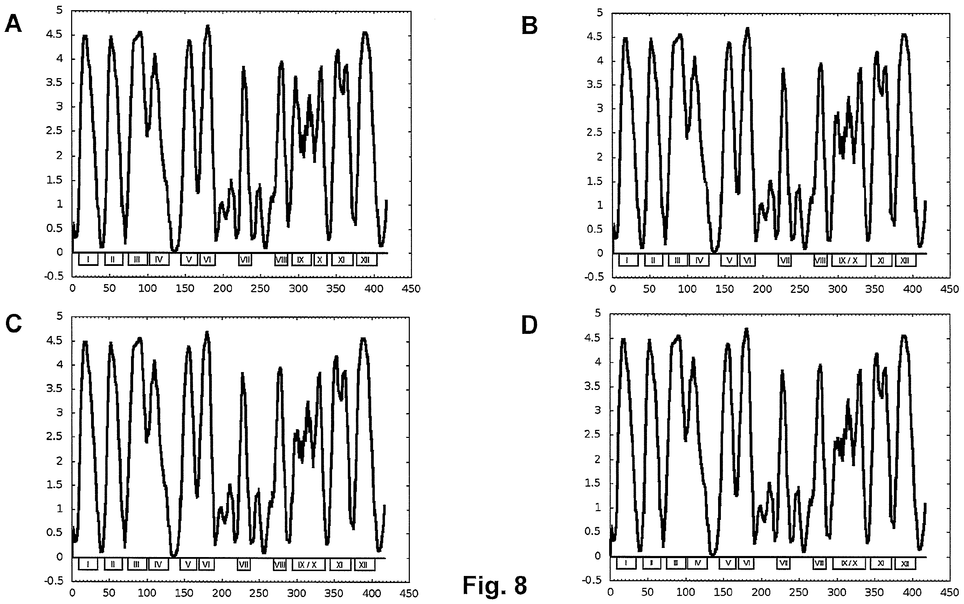

Hydrophobicity of *E*. *coli* wild-type LacY is shown in Fig. 8 A and Fig. 9 A, and assessment of the hydrophobicity revealed the presence of twelve transmembrane domains within the lactose permease as indicated by boxes I to XII below the curve. While assessment of hydrophobicity of the variant LacY(L293H) showed no alteration of the number of transmembrane domains (Fig. 9 B), it was found that *E*. *coli* LacY variants LacY(L293R) (Fig. 9 C) and LacY(L293K) (Fig. 9 D), as well as *E*. *coli* LacY variants LacY(L292R) (Fig.8 B), LacY(L294R) and LacY(L292R), LacY(L293R), LacY(L294R) (Fig. 10) display an altered hydrophobicity which leads to the fusion of transmembrane domains IX and X - with reference to *E. coli* wild-type LacY - to a single transmembrane domain such that periplasmic loop P9 (Fig. 2) is not accounted for in the prediction of transmembrane domains. However, the predicted alteration of the LacY variants' membrane topology - as compared to the membrane topology of *E*. *coli* wild-type LacY - does not fully abolish transmembrane transport of lactose by the LacY variants.

## Claims

1. A genetically engineered microbial cell for the production of an oligosaccharide of interest, wherein said microbial cell comprises a lactose permease for internalizing exogenous lactose, wherein said lactose permease is a variant of the *E. coli* lactose permease LacY which exhibits a reduced internalization of lactose as compared to *E. coli* wild-type LacY.

2. The genetically engineered microbial cell according to claim 1, wherein the variant of the *E. coli* lactose permease LacY has a sequence identity of at least 70 %, at least 80 %, at least 90 %, at least 95 % sequence identity to *E. coli* wild-type lactose permease LacY.

3. The genetically engineered microbial cell according to claim 1 or 2, wherein the variant of the *E. coli* lactose permease LacY comprises a basic amino acid residue in at least one of amino acid positions 292, 293 and 293 with respect to the lactose permease as represented by SEQ ID No. 1.

4. The genetically engineered microbial cell according to claim 3, wherein the basic amino acid residue is selected from the group consisting of arginine, histidine and lysine.

5. The genetically-engineered microbial cell according to any one of claims 1 to 4, wherein the microbial cell comprises a nucleic acid molecule comprising a nucleotide sequence from the group consisting of
a) nucleotide sequences encoding a variant of the *E*. *coli* wild-type lactose permease LacY as defined in any one of claims 1 to 4,
b) nucleotide sequences which are complementary to any one of the nucleotide sequences of a);
c) nucleotide sequences that hybridize to any one of the nucleotide sequences set forth in a) or b) under stringent conditions.

6. The genetically engineered microbial cell according to claim 5, wherein the nucleotide sequence encoding a variant of the *E. coli* wild-type lactose permease LacY is operably inked to expression control sequences.

7. The genetically engineered microbial cell according to any one of claims 1 to 6, wherein the microbial cell further comprises
- at least one metabolic pathway for intracellular biosynthesis of a nucleotide activated sugar; and
- at least one glycosyltransferase for transferring intracellularly a monosaccharide moiety from the nucleotide-activated monosaccharide as donor substrate to lactose or an oligosaccharide as acceptor molecule.

8. Use of a genetically engineered microbial cell according to any one of claims 1 to 7 for the production of an oligosaccharide of interest, wherein said oligosaccharide of interest comprises a lactose moiety at its reducing end.

9. A method for producing an oligosaccharide of interest, wherein the oligosaccharide of interest is synthesized in a microbial cell, the method comprises:
- providing a genetically engineered microbial cell for intracellular biosynthesis of the oligosaccharide as defined in any one of claims 1 to 7;
- cultivating the genetically engineered cell in the presence of exogenous lactose in a medium and under conditions allowing the cell to intracellularly synthesize the oligosaccharide of interest; and
- recovering the oligosaccharide of interest from the culture medium or the cell.

10. The method according to claim 9, wherein the oligosaccharide is selected from the group consisting of 2'-fucosyllactose (2'-FL), 3-fucosyllactose (3-FL), 2',3-difucosyllactose (DFL), lacto-*N*-triose II, lacto-*N*-tetraose (LNT), lacto-*N*-neotetraose (LNnT), lacto-*N*-fucopentaose I (LNFP-I), lacto-*N*-*neo*fucopentaose I (LN*n*FP-I), lacto-*N*-fucopentaose II (LNFP-II), lacto-*N*-fucopentaose III (LNFP-III), lacto-*N*-fucopentaose V (LNFP-V), lacto-*N*-*neo*fucopentaose V (LNnFP-V), lacto-*N*-hexaose (LNH), lacto-*N*-neohexaose (LNnH), para-lacto-N-hexaose (paraLNH), para-lacto-N-neohexaose (paraLNnH), difucosyl-lacto-N-neohexaose (DF-LNnH), lacto-*N*-difucosylhexaose l, lacto-*N*-difucosylhexaose II, *para-*lacto-*N*-fucosylhexaose (paraLNH), fucosyl-lacto-*N*-sialylpentaose a (F-LST-a), fucosyl-lacto-*N*-sialylpentaose b (F-LST-b), fucosyl-lacto-*N*-sialylpentaose c (F-LST-c), fucosyl-lacto-*N*-sialylpentaose c, disialyl-lacto-*N*-fucopentaose, 3-fucosyl-3'-sialyllactose (3F-3'-SL), 3-fucosyl-6'-sialyllactose (3F-6'-SL), lacto-N-neodifucohexaose l, 3'-sialyllactose (3-SL), 6'-sialyllactose (6-SL), sialyllacto-*N*-tetraose a (LST-a), sialyllacto-*N*-tetraose b (LST-b), sialyllacto-*N*-tetraose c (LST-c), disialyllacto-*N*-tetraose (DS-LNT), Disialyl-lacto-*N*-fucopentaose (DS-LNFP V), lacto-N-neodifucohxaose I (LNnDFH I), 3'-galactosyllactose (3'-GL), 6'-galactosyllactose (6'-GL).
